# EUROPEAN PATENT APPLICATION

(11) **EP 1 479 777 A1**
(43) Date of publication of application: **24.11.2004**
(21) Application number: 03702662.2
(22) Date of filing: 03.02.2003
(51) Int. Cl.: C12P 23/00, C12N 1/16, A23K 1/18, A23L 1/28

(54) **METHOD OF PRODUCING ASTAXANTHIN BY FERMENTING SELECTED STRAINS OF XANTHOPHYLLOMYCES DENDRORHOUS**

(30) Priority: 04.02.2002 ES 200200257
(71) Applicant: Vitatene, S.A., 24080 Leon (ES)
(72) Inventor: DE LA FUENTE MORENO, Juan, Luis, E-24009 León (ES); PEIRO CEZON, Enrique, E-24009 León (ES); DIEZ GARCIA, Bruno, E-24009 León (ES); MARCOS RODRIGUEZ, Ana, Teresa, E-24009 León (ES); SCHLEISSNER SANCHEZ, Carmen, E-24009 León (ES); RODRIGUEZ SAIZ, Marta, E-24009 León (ES); RODRIGUEZ OTERO, Carmelita, E-24009 León (ES); CABRI, Walter, E-24009 León (ES); BARREDO FUENTE, José, Luis, E-24009 León (ES)
(74) Representative: Elzaburu Marquez, Alberto
(86) International application number: PCT/ES2003/000063
(87) International publication number: WO 2003/066875

(57) **Abstract**

The present invention describes methods for (i) obtaining astaxanthin-overproducing strains of *X*. *dendrorhous* and (ii) the use of the aforementioned strains in improved conditions of fermentation. The methods used for selecting the strains are based on: (i) resistance to inhibitors of steroid synthesis, to inhibitors of respiration and to compounds that induce the formation of free radicals, (ii) color intensity of the colony and production of carotenoids on solid medium, (iii) production of astaxanthin in darkness, (iv) production of astaxanthin in conditions with a raised temperature and (v) production of astaxanthin with carbon sources other than glucose. These methods permit the efficient selection of mutants of *X*. *dendrorhous* with astaxanthin yields higher than the parent strain and little accumulation of other carotenoids. Cultivation of *X*. *dendrorhous* in liquid medium makes it possible to obtain a biomass that can be used directly in human and animal nutrition.

## Description

### Field of the invention

The present invention describes methods for (i) selecting astaxanthin-overproducing strains of *Xanthophyllomyces dendrorhous* and (ii) using the aforementioned strains in improved conditions of fermentation.

### State of the art

The carotenoids are pigments of isoprenoid nature that are synthesized by certain bacteria, fungi and photosynthetic organisms. Owing to their beneficial effects on health and their attractive colors, the carotenoids are of great commercial importance as colorants and food additives. The carotenoids are polyene compounds with 40 or 50 carbon atoms formed from 8-10 isoprene units (C5). They have an absorption maximum between 400 and 500 nm, which gives them their characteristic coloration between yellow and red. Carotenoids with an unsubstituted hydrocarbon chain (lycopene, β-carotene) are called carotenes. The oxygenated derivatives are called xanthophylls. The latter include alcohols (lutein and zeaxanthin), epoxides (violaxanthin), esters (spheroidene), ketones (equinenone, canthaxanthin, astaxanthin) and acids (torularhodin). The presence of the chromophore group in the structure of the carotenoids has an important biological function as photoprotective agent and antioxidant. The carotenoids protect vulnerable tissues against the effects of oxygen singlets and free radicals. In this way, the biosynthetic intermediate of astaxanthin, 3-hydroxy-3',4'-didehydro-β,ψ-caroten-4-one (HDCO), performs an important function as a free radical trap, as well as imparting a very pleasing reddish pigmentation. The carotenoids perform numerous biological functions, and in particular they act as anticancer agents, potentiators of the immune system, agents that prevent degenerative diseases of the central nervous system (Alzheimer's, diseases associated with vision, etc.), anti-inflammatory agents, antistress agents, photoprotective agents, etc. Astaxanthin is a ketocarotenoid (xanthophyll) that is widely distributed in nature, whose molecule has eleven conjugated double bonds. It is produced by bacteria (*Agrobacterium aurantiacum, Rhodococcus maris*, etc.), fungi (*X. dendrorhous*), algae (*Haematococcus pluvialis*) and higher plants. In general, all the green parts of plants contain astaxanthin masked by chlorophyll, which, along with other carotenoids, endows them with the typical autumnal coloration. It is also found in flowers of *Adonis aestivalis,* in birds (flamingo and scarlet ibis), fish (salmonids) and marine invertebrates (shrimp, crab, lobster, etc.) and fulfils important functions for the protection and survival of these living beings.

The production of carotenoids by microbial biosynthesis is a classic example of competition between chemical and biological processes. The biotechnological processes display, among other things, the advantage that they make it possible to obtain, in a simple manner, the carotenoids of more complex structure, as well as the conformational isomers that only exist naturally. The industrial biotechnological processes for the production of astaxanthin, competing with chemical synthesis, are based on the use of the alga *H. pluvialis* and of the yeast *X*. *dendrorhous.* The industrial production of astaxanthin with *H*. *pluvialis* involves the need for extensive areas of sea for its cultivation, the existence of contamination and the difficulty of controlling certain environmental factors. Therefore, when *X*. *dendrorhous* was described for the first time in 1976 (Phaff H.F. et al. 1972. Proc. IV IFS: Ferment. Technol. Today 759-774) and its ability to produce astaxanthin and other carotenoids was demonstrated (Johnson et al. 1978. Appl. Environ. Microbiol. 35: 1155-1159), as well as its efficacy in the pigmentation of salmonids, birds' eggs, etc., the yeast quickly attracted increasing industrial interest.

The astaxanthin-rich biomass of *X*. *dendrorhous* can be used as a food additive for coloring the meat from salmonids. Salmon acquire their typical coloration in their natural environment because their food includes microorganisms and crustaceans that contain astaxanthin. Fish grown in captivity originally had an off-white coloration of the flesh and skin, owing to the absence of astaxanthin in their food. Astaxanthin, as well as imparting color and flavour, plays an important role in reproduction and in the general development of the fish. At present, most astaxanthin is obtained by chemical synthesis, since its extraction from the shells of crustaceans is not economically profitable. However, owing to increasing consumer sensitivity to chemical additives and synthetic products, the use of *X*. *dendrorhous* and *H*. *pluvialis* as natural sources of astaxanthin is increasing.

The biosynthetic pathway of astaxanthin (see Scheme 1) has been described in a number of organisms such as *X*. *dendrorhous, Erwinia uredovora* and *Agrobacterium aurantiacum* (Ducrey Sanpietro L.M. 1998. Yeast 14: 1007-1016; Misawa N. et al. 1995. J. Bacteriol. 177: 6575-6584; Fraser P.D. et al. 1997. J. Biol. Chem. 272: 6128-6135). At least five enzymes are required for this biosynthesis: (i) phytoene synthase, which joins together two molecules of geranylgeranyl pyrophosphate to generate phytoene, (ii) phytoene dehydrogenase, which introduces four double bonds in the phytoene molecule to synthesize lycopene, (iii) lycopene cyclase, which, using lycopene as substrate, forms the rings situated at both ends of the β-carotene molecule, (iv) β-carotene ketolase, which catalyses the introduction of a keto group in each of the rings situated at the two ends of the β-carotene molecule and (v) β-carotene hydroxylase, which performs hydroxylation of each of the rings situated at each end of the β-carotene molecule. These last two enzymes take part in the biosynthesis of a series of precursors (equinenone, canthaxanthin, phoenicoxanthin, β-cryptoxanthin, zeaxanthin, adonixanthin) of astaxanthin (Scheme 1). The enzymes β-carotene ketolase and β-carotene hydroxylase (as well as the genes encoding them) have been characterized in a number of organisms such as *E*. *uredovora, A. aurantiacum, Arabidopsis thaliana*, etc. and in all cases the existence of two separate proteins has been described. However, in *X*. *dendrorhous* the existence of a single enzyme called astaxanthin synthase has been described, catalysing the conversion of β-carotene to astaxanthin (Tatsuo H. et al. 2000. EP1035206). 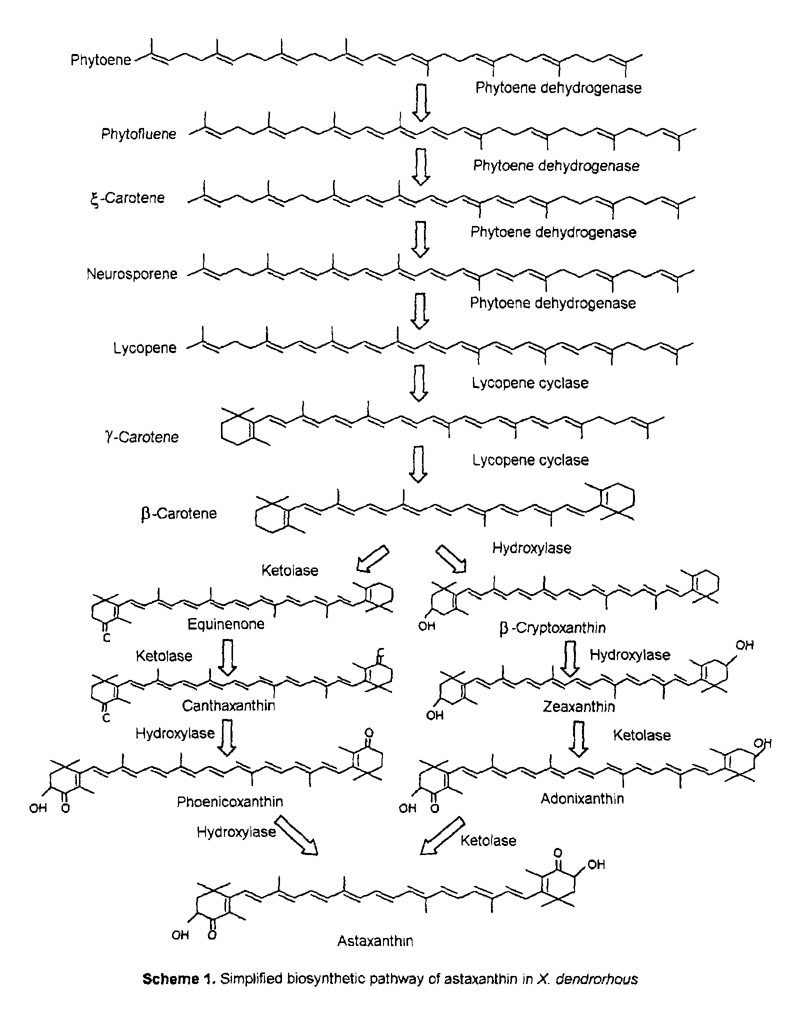 The growing commercial interest in *X*. *dendrorhous* for the production of astaxanthin has promoted the application of molecular typing techniques in this microorganism (Adrio J.L. et al. 1995. Curr. Genet. 27: 447-450). In this way it is possible to establish differences between the strains selected in improvement programmes. In addition, these techniques can be used for controlling the quality of the biomass obtained, since they make it possible to determine the genetic stability of the strain during fermentation and the presence of contamination by other microorganisms.

The use of DNA probes is indicated for the analysis of microorganisms for which suitable probes exist and which cannot be differentiated easily by biochemical methods. DNA amplification is the method used for direct detection, owing to its sensitivity and speed. In the majority of cases the amplified DNA fragment is detected simply by staining the agarose gel with ethidium bromide. The molecular typing techniques based on DNA electrophoresis include the following: RFLP (Restriction Fragment Length Polymorphism), ribotyping (hybridization with rRNA probes), PFGE (Pulsed Field Gel Electrophoresis), OFAGE (Orthogonal Field Gel Electrophoresis), FIGE (Field Inversion Gel Electrophoresis), CHEF (Clamped Homogeneous Electrical Field), etc. On the other hand there are several methods of molecular typing based on DNA amplification: PCR (Polymerase Chain Reaction), PCR-RFLP, REP (Repetitive Extragenic Palindromic), ERIC (Enterobacterial Repetitive Intergenic Consensus), RAPD (Randomly Amplified Polymorphic DNA), etc. The RFLP electrophoresis technique has been much used for the detection of changes in the DNA of various organisms. However, the discovery of the PCR technique meant that methods such as RAPD provide quick and efficient alternatives to the electrophoresis techniques. In addition, RAPD has the advantage that it does not require the use of radioactive isotopes and that it needs smaller quantities of DNA for the analysis. This technique is based on the use of a short oligonucleotide as primer. The said oligonucleotide can bind to different regions of the genomic DNA used as template, making it possible to amplify particular DNA sequences. The primers used in RAPD possess an arbitrary sequence, normally with a G+C content greater than 50% and absence of repeated inverted internal sequences. The theoretical number of amplified DNA fragments depends on the length of the primer and on the size of the genome employed as template. Amplification is based on the probability that: (i) the primer finds a complementary DNA sequence in the genome, (ii) the said sequence is located on opposite strands, (iii) it appears in the inverted sense and (iv) at a distance that can be amplified by PCR. The appearance of polymorphisms may be due to changes in the binding sequence of the primer (for example, point mutations) which prevent correct binding of the primer to the template DNA. Furthermore, these polymorphisms may be caused by changes in the DNA sequence (e.g. insertions and inversions) which alter the size of the amplified fragment, prevent amplification of the DNA because they give rise to primer binding sites that are too remote, cause deletions of the primer binding site, etc.

The methods of selecting strains used in the present invention are based on: (i) resistance to inhibitors of the synthesis of steroids, to inhibitors of respiration and to compounds that induce the formation of free radicals, (ii) color intensity of the colony and production of carotenoids on solid medium, (iii) production of astaxanthin in darkness, (iv) production of astaxanthin in conditions of elevated temperature and (v) production of astaxanthin with carbon sources other than glucose. These methods permit efficient selection of mutants of *X*. *dendrorhous.* Moreover, the methods for selection of strains and fermentation described in the present invention make it possible to achieve high yields of astaxanthin and low levels of accumulation of other carotenoids. Cultivation of *X*. *dendrorhous* in a liquid medium makes it possible to obtain nutritive, health-promoting biomass owing to its content of carotenoids, proteins, carbohydrates, vitamins, fatty acids and other nutrients. The said biomass can be used directly in food for humans and animals.

### Detailed description of the invention

The present invention describes a number of methods for obtaining high yields of astaxanthin using the yeast *X*. *dendrorhous.* The invention comprises (i) the design of methods for obtaining and selecting astaxanthin-overproducing mutants of *X*. *dendrorhous* and (ii) the development of improved conditions of fermentation. Research was directed towards obtaining strains of *X*. *dendrorhous* with a higher concentration of astaxanthin. This was based on applying the classical techniques of mutation and screening, and optimizing both the raw materials and the conditions of fermentation. The concentration of astaxanthin is expressed as (i) ppm (µg of pure astaxanthin per g of dry biomass) or (ii) percentage of pure astaxanthin relative to dry biomass. Whereas the wild-type strains of *X*. *dendrorhous* produce between 100 and 200 ppm of astaxanthin, the present patent describes methods for the production of at least 5000 ppm. *X*. *dendrorhous* is of great industrial importance for the biotechnological production of astaxanthin. In fact, the said process is competitive with the synthetic method used industrially at present.

With the aim of obtaining astaxanthin-overproducing strains, firstly a mutagenic method was developed for strains of *X*. *dendrorhous* with the mutagenic agents ethylmethane sulphonate (EMS), N-methyl-N'-nitro-N-nitrosoguanidine (NTG) and ultraviolet radiation (UVA). Suspensions of cells to be mutated were obtained from a culture of *X*. *dendrorhous* in the liquid medium R4-062-7. The method of mutation with EMS consisted of incubating 10⁸ cells/ml in a 6% EMS solution in 0.1M sodium phosphate buffer pH 7.0 at 20°C and 100 rpm for 40-80 minutes. The method of mutation with NTG consisted of incubating 10⁸ cells/ml in a solution of 250 µg/ml of NTG in 0.1M sodium citrate buffer pH 5.0 at 20°C and 100 rpm for 60-120 minutes. The method of mutation with UVA consisted of irradiating, with a 254 nm lamp, a suspension of 10⁷ cells/ml in saline solution at 20°C and 40 rpm for 5-10 minutes. In the three methods of mutation employed, the mutated cells were incubated for 10 hours at 17-20°C and 100 rpm in YEPD liquid medium to promote their recovery. Next, Petri dishes containing YEPDA solid medium were seeded, and were incubated at 17°C for 4 days to obtain isolated colonies.

The strategies used for selecting astaxanthin-overproducing strains of *X*. *dendrorhous* were as follows: (i) resistance to inhibitors of the synthesis of steroids, to inhibitors of respiration and to compounds that induce the formation of free radicals, (ii) color intensity of the colony and production of carotenoids on solid medium, (iii) production of astaxanthin in darkness, (iv) production of astaxanthin in conditions of elevated temperature and (v) production of astaxanthin using carbon sources other than glucose. For selecting astaxanthin-overproducing mutants, the mutated cells were grown on YEPDA medium to which the following had been added: (i) compounds that alter the redox potential of the cell such as duroquinone or hydrogen peroxide or (ii) inhibitors of steroid synthesis, especially β-ionone, imidazole, diethylamine, 2-methylimidazole, nystatin and diphenylamine.

The mutants were selected subsequently as a function of their yield on solid medium. The said selection was performed in a first phase by isolating those colonies that had a deeper red coloration than the parent strain and then using a method of evaluating the carotenoid content of the biomass grown on solid medium. By selecting the mutants that had an absorbance value greater than that of the parent strain VKPM Y-2476, it was possible to select the astaxanthin-overproducing strains AST-A1 and AST-A2 (Scheme 2). The mutants that were astaxanthin producers in darkness were selected as a function of their carotenoid content when incubated on solid medium in darkness. The strains AST-A3, AST-A4, AST-A5, AST-A6 and AST-A7 were selected in this way (Scheme 2). The mutants that were producers of astaxanthin in conditions of elevated temperature were selected as a function of their ability to grow and produce astaxanthin at temperatures above the usual temperatures (17-20°C). The strains AST-A8, AST-A9 and AST-A10 (Scheme 2) were selected in this way, for their ability to grow at 24°C and their yield of astaxanthin. The strains of *X*. *dendrorhous* capable of producing astaxanthin with carbon sources other than glucose were selected as a function of their ability to grow and produce astaxanthin in the presence of sucrose as the sole source of carbon. In this way the strain AST-A11 was selected (Scheme 2), which exhibited a capacity for production of astaxanthin greater than that of the parent strain when sucrose was used as the source of carbon.

The strains selected were submitted to a number of genetic analyzes with the aim of establishing differential characteristics. In this way the existence of extrachromosomal elements was demonstrated in the strains of *X*. *dendrorhous.* In addition it was confirmed (i) that the said extrachromosomal elements (plasmids) were made up of double-stranded DNA of linear conformation and (ii) that the astaxanthin-overproducing strains possessed a pattern of extrachromosomal elements different from that exhibited by the strains with lower production. The differences established between the astaxanthin-overproducing strains and the strains with lower production were confirmed additionally using the RAPD technique.

The selected strains were fermented in a flask for the purpose of determining the yield of astaxanthin in liquid medium. For this, flasks of inoculum were grown and then flask fermentation was carried out. On completion of fermentation (about 7 days), *X*. *dendrorhous* was lysed using vortex agitation, the carotenoids produced were extracted with organic solvents (e.g. with acetone) and the concentration and purity of the astaxanthin were determined by HPLC. The yields obtained varied between 150 and 200 mg/l. Adding the following to the culture medium: (i) agents that release free radicals, such as duroquinone, (ii) compounds that are inducers of carotenogenesis such as retinal or trisporic acids or (iii) precursors of the hydrocarbon chain such as glutamate, improved the yields of astaxanthin, with yields reaching at least 225 mg/l with retinal, 200 mg/l with trisporic acids and 180 mg/l with glutamate. In addition, (i) a culture medium was developed that increased the specific yield of astaxanthin and (ii) it was confirmed that both white light and ultraviolet light increased the yield of astaxanthin, the best results being obtained with cycles of 6 hours of ultraviolet light / darkness.

The strains selected in the flask were cultivated in pilot-plant fermenters with the aim of determining the yield of astaxanthin. The fermentation conditions included initial growing of the microorganism on complex culture media that make it possible to obtain a high level of biomass and a maximum yield. The initial growing stage was followed by a production phase characterized by minimum growth and maximum yield. In addition, the yield of astaxanthin was increased by the following methods: (I) Addition of substances with oxidizing power to the culture medium (for example duroquinone at a concentration of 25-50 µM) which induce the intracellular formation of free radicals. Addition must be effected during the first 24 hours of fermentation to be really effective. These compounds reduce growth (15-25%) and increase the yield of astaxanthin (10-20%). Therefore the overall increase in specific productivity of the biomass can vary in the range 30-60%, reaching astaxanthin yields of at least 215 mg/l in 6-7 days of fermentation in a flask. (II) Reduction of the additions of the easily assimilated carbon source (e.g. glucose) in an advanced phase of fermentation and partial replacement with a carbon source with slower utilization (e.g. ethanol, glycerol, etc.) which makes it possible to maintain growth that is less vigorous but is sufficient for maintenance of the productive biomass. (III) Illumination of the culture in the production phase with high-intensity light (for example above 250 lux). (IV) Change of the values of pH and temperature to conditions that favour yield over growth. At temperatures around 17°C and pH values close to 3, growth is slowed and the yield is increased. The combined effect of these variables made it possible to increase the yield of astaxanthin and obtain an increased level of biomass. This means a higher yield per unit of fermenter volume, one of the most important production objectives from the industrial standpoint.

The fermentation process was carried out in a culture medium that contains sources of carbon, sources of nitrogen, mineral salts and vitamins (e.g. thiamine, biotin, calcium pantothenate, etc.). Nutrients that are rich in carbohydrates can be used as sources of carbon, such as dextrins, starches, glucose, sucrose, fructose or vegetable flours that are rich in some of these sugars. The following can be used as sources of nitrogen: (i) organic compounds such as yeast extract, cottonseed flour (Pharmamedia), corn steep, soybean flour, peptones, casein, etc. or (ii) inorganic compounds such as ammonium sulphate, diammonium phosphate, etc. The mineral salts that can be added to the culture medium include phosphates, sulphates or chlorides of monovalent cations (sodium, potassium, ammonium, etc.) or divalent cations (calcium, magnesium, etc.). Furthermore, certain trace elements can be added to the culture medium, such as Cu, I, Fe, Mn, Mo, Zn, etc. The proportions of the nutrients were determined on the basis of the growth requirements of the microorganism and the yields. Fermentation was carried out in submerged aerobic culture at a temperature between 17°C and 22°C, except when using mutants capable of producing astaxanthin at 24°C. The pH of the culture was allowed to vary freely during the first few hours and was then controlled in the range 3.0-5.0 by adding alkali. The start of pH control depends on the development of growth, and generally begins during the first 24 hours of fermentation. The process was characterized by high initial growth based on the availability of the sources of carbon and nitrogen, aeration greater than 1 v/v/m (volume/volume/minute) and a speed of stirring that ensured a level of dissolved oxygen above 50% after completion of the initial growth phase. Growth of *X*. *dendrorhous* was maintained with additions of the source of carbon (generally glucose) so as to achieve the maximum concentration of biomass with high specific yield. Achievement of this objective gives higher productivity per fermenter installed volume relative to the technologies developed previously.

With the strategies described above, it was possible to obtain fermentation media with biomass above 70 g/l and a yield of at least 400 mg/l, which means astaxanthin yields per biomass unit above 5000 ppm. The biomass obtained comes from new strains of *X*. *dendrorhous*, which can be differentiated from other strains of the same species using methods of molecular typing. In addition to the selection of astaxanthin-overproducing strains, the fermentation technology has been optimized to make it more profitable industrially. Despite the fact that yields of biomass of *X*. *dendrorhous* have been described with a high astaxanthin content, in these cases there is very limited growth of the yeast in the culture medium (5-30 g/l). This means that the volume of culture medium that must be processed to obtain a large quantity of carotenoids is very high. Our results provide a new fermentation process which gives not only high yields of carotenoids, but also a concentration of yeasts in the fermentation culture of 50-100 g/l. These results provide an important advantage for industrial productivity and reduction of production costs. 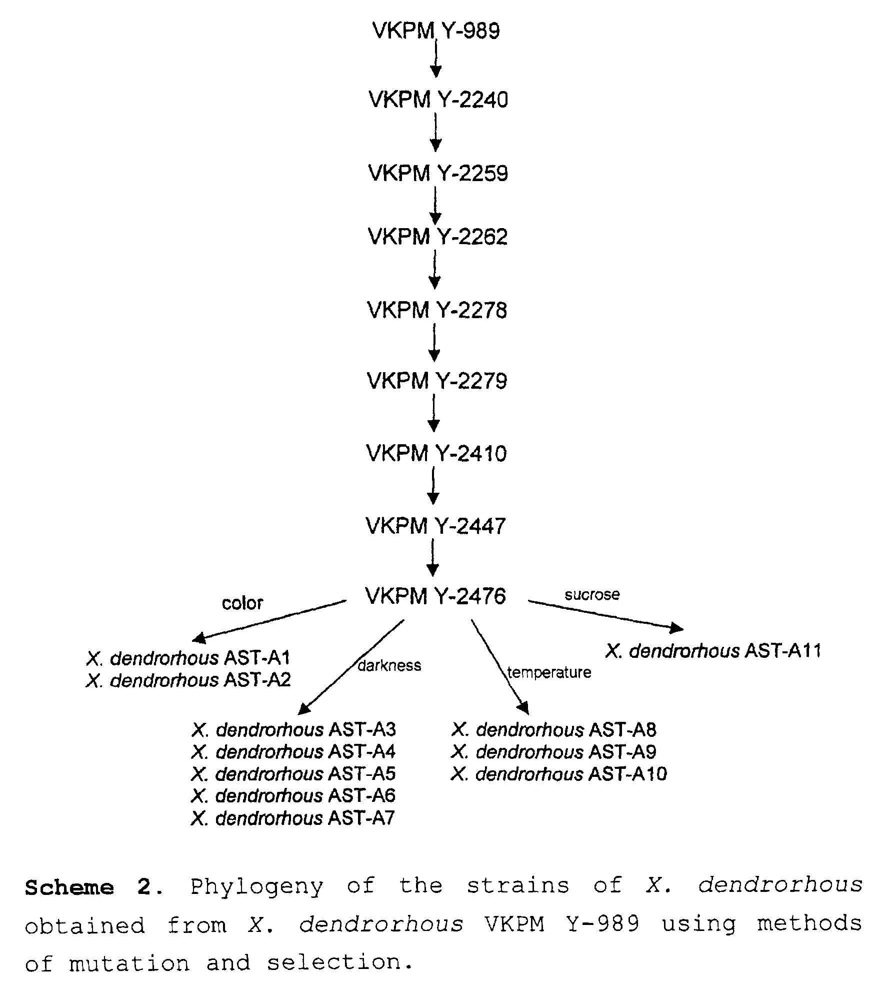 The biomass was separated from the culture medium by centrifugation or filtration, it was dried and was then used for carrying out a series of tests of bioavailability in trout. For this, the said dry biomass was added to the feed for the trout and this mixture was fed to a number of trout for 2 months. Finally, the presence of astaxanthin and carotenoids in the tissue of 10 of the said trout was determined. The results obtained (3.5 µg/g of astaxanthin and 6.8 µg/g of carotenoids) demonstrated the ability of the *X*. *dendrorhous* biomass to impart a salmon-colored pigmentation to the trout.

### Deposit of Microorganisms in accordance with the Treaty of Budapest

The strains of *X*. *dendrorhous* have been deposited, in accordance with the provisions of the Treaty of Budapest, in the Russian National Collection of Industrial Microorganisms (VKPM), GNII Genetika, Dorozhny Proezd 1, Moscow 113545 (Russia), with the following numbers and dates: VKPM Y-989 on 13/03/1989, VKPM Y-2240 on 20/12/1996, VKPM Y-2259 on 31/01/1997, VKPM Y-2262 on 10/06/1997, VKPM Y-2278 on 06/08/1997, VKPM Y-2279 on 31/10/1997, VKPM Y-2410 on 30/10/1998, VKPM Y-2447 on 22/09/1999 and VKPM Y-2476 on 06/03/2000.

The following examples describe the present invention in detail and without limitation.

### EXAMPLE 1

### Strategies for mutation of X. dendrorhous

Firstly a mutagenic method was developed for the strains of *X*. *dendrorhous,* for which the following were analyzed: (i) different types of mutagenic agents, (ii) concentration of the mutagen, (iii) concentration of cells, (iv) incubation pH and (v) treatment time. In this way, the following were selected as mutagenic agents: ethylmethane sulphonate (EMS), N-methyl-N'-nitro-N-nitrosoguanidine (NTG) and ultraviolet radiation (UVA).

The suspensions of cells to be mutated were obtained by seeding a liquid culture in 500-ml flasks with 25 ml of R4-062-7 medium and incubating it for 24 hours at 17-20°C and 250 rpm. The R4-062-7 medium has the following composition: 6.2 g/l of yeast extract, 5.5 g/l of cottonseed flour, 70 g/l glucose, 2 g/l KH₂PO₄, 0.4 g/l K₂HPO₄, 1.5 g/l MgSO₄·7H₂O, 2 g/l (NH₄)₂SO₄, 0.2 g/l NaCl, 0.2 g/l CaCl₂, 50 µg/l biotin, 500 µg/l thiamine and 2 mg/l calcium pantothenate, at a final pH of 5.8-6.0. The concentration of cells in the suspension was about 10⁸ cells/ml. These cells were washed twice with saline solution, centrifuging at 3000 rpm and 10°C for 3 minutes, and then their concentration was adjusted to 2 x 10⁸ cells/ml.

The method of mutation with EMS consisted of incubating 10⁸ cells/ml in a 6% EMS solution in 0.1M sodium phosphate buffer pH 7.0 at 20°C and 100 rpm for 40-80 minutes, achieving mortality rates of 90-99%. The method of mutation with NTG consisted of incubating 10⁸ cells/ml in a solution that contained 250 µg/ml of NTG in 0.1M sodium citrate buffer pH 5.0 at 20°C and 100 rpm for 60-120 minutes, achieving mortality rates of 90-99%. The mutated cells were washed three times with saline solution, centrifuged at 3000 rpm and 10°C for 3 minutes, and then they were cultivated in liquid medium to promote their recovery. For this, they were resuspended in 10 ml of YEPD medium and were incubated in 250-ml flasks for 10 hours at 17-20°C and 100 rpm. The composition of the YEPD medium is as follows: 20 g/l of bactopeptone, 10 g/l of yeast extract and 20 g/l of glucose, at a final pH of 6.0.

The method of mutation with UVA consisted of treating a suspension of 10⁷ cells/ml in saline solution with the radiation from a 254 nm lamp at 20°C and 40 rpm for 5-10 minutes, achieving mortality rates of 90-99%. The mutated cells were left at rest in darkness for 30 minutes and were then cultivated in liquid medium for their recovery. For this, they were resuspended in 10 ml of YEPD and were incubated in darkness in 250-ml flasks for 10 hours at 17-20°C and 100 rpm.

The mutated cells were used for seeding Petri dishes that contained YEPDA solid medium, and were incubated at 17°C for 4 days to obtain isolated colonies. The composition of the YEPDA medium is as follows: 20 g/l bactopeptone, 10 g/l yeast extract, 20 g/l glucose and 20 g/l agar, at a final pH of 6.0. The seeded dishes were incubated at 17°C for 4 days to obtain isolated colonies.

### EXAMPLE 2

### Strategies for selecting astaxanthin-overproducing strains of X. dendrorhous as a function of the yield of carotenoids on solid medium

The colonies obtained from the mutated cells described in example 1 were seeded directly on YEPDA medium or on YEPDA medium to which the following had been added: (i) compounds that alter the redox potential of the cell or (ii) inhibitors of the synthesis of steroids. Among the compounds of type (i), in particular duroquinone (100 µM) and hydrogen peroxide (5 mg/l) were used, whereas among those of type (ii), the following were used in particular: β-ionone (50 µl/l), imidazole (5 mM), diethylamine (10 µM), 2-methylimidazole (5 mM), nystatin (1 mg/l) and diphenylamine (100 µM).

The colonies arising from the aforementioned cultures were seeded again on YEPDA medium in the form of stripes of about 3 cm² and were incubated at 17-20°C for 4 days in the presence of light. In a first phase of the strain selecting programme it was possible to isolate a number of stripes that exhibited greater red coloration than the parent strain. Subsequently, the deep red coloration of the parent strains prevented direct selection based on color, and a method was developed for determination of carotenoids from the biomass grown on solid medium. This method consisted of removing, from each stripe, the biomass corresponding to 0.8 cm² and resuspending it in 1 ml of dimethylsulphoxide. The carotenoids were extracted using vortex agitation for 2 minutes and centrifugation at 12 000 rpm for 5 minutes at room temperature. The yield of carotenoids was evaluated by measuring the absorbance of the supernatant at 474 nm. Selecting the strains that had an absorbance value greater than that of the VKPM Y-2476 parent strain made it possible to select the astaxanthin-overproducing mutants AST-A1 and AST-A2.

### EXAMPLE 3

### Strategies for selecting strains of X. dendrorhous that are astaxanthin overproducers in darkness

The method consisted of evaluating the mutants for their capacity to grow and produce astaxanthin in the absence of light. For this, a series of YEPDA dishes were seeded with a suspension of mutated cells and were incubated at 22°C for 4 days in the absence of light. Then a total of 103 colonies that exhibited greater coloration and growth were selected. These colonies were seeded again on YEPDA in the form of stripes of about 3 cm² and were incubated at 22°C for 4 days in the absence of light. The biomass corresponding to 0.8 cm² of each stripe was used for seeding 50-ml flasks containing 10 ml of R4-062-7 medium, which were incubated for 72 hours at 20°C and 250 rpm in the absence of light. From these cultures, 5 mutants were selected (AST-A3, AST-A4, AST-A5, AST-A6 and AST-A7) with higher yield of carotenoids, and these were tested in fermentations both in darkness and in the presence of light, it being observed that the astaxanthin yield was twice that of the parent strain VKPM Y-2476 in darkness and was equivalent in the presence of light.

### EXAMPLE 4

### Strategies for selecting strains of X. dendrorhous that are astaxanthin overproducers in conditions of elevated temperature

The method consisted of analysing the mutants for their capacity to grow and produce astaxanthin at temperatures above the usual temperatures for *X*. *dendrorhous* (17-20°C). The programme was carried out in two phases: firstly at 22°C and then at 24°C. For this, a suspension of cells mutated with NTG originating from the VKPM Y-2476 strain was seeded on dishes of YEPDA, which were incubated at 22°C for 6 days in the absence of light. In these conditions the VKPM Y-2476 parent strain is unable to grow. In this way, 100 colonies were isolated based on their ability to grow at 22°C. These colonies were seeded again on YEPDA in the form of stripes of about 3 cm² and were incubated at 22°C for 4 days in the presence of light. Then the yield of carotenoids on solid medium was evaluated and a total of 10 strains were selected, and these were fermented in liquid medium both at 20°C and at 22°C. Finally, 4 strains were preselected.

A combined cellular suspension from the 4 strains previously selected was submitted to mutation with NTG as indicated in example 1, seeding the mutated cells on YEPDA and then incubating the dishes at 23.5°C for 6 days in the absence of light. In this way 200 colonies were selected, and these were seeded again on YEPDA in the form of stripes of about 3 cm² and were incubated at 24°C for 4 days in the presence of light. Next, the yield of carotenoids on solid medium was evaluated, selecting a total of 6 strains. Once the astaxanthin yield of these 6 strains had been analyzed at 24°C in liquid medium, 3 of them were selected, and were designated AST-A8, AST-A9 and AST-A10.

### EXAMPLE 5

### Strategies for selecting strains of X. dendrorhous capable of producing astaxanthin with carbon sources other than glucose

The colonies obtained from the mutated cells described in example 1 were seeded directly on YEPDA medium to which glucose (20 g/l) or sucrose (20 g/l) had been added. The colonies originating from the cultures previously mentioned were seeded again on YEPDA-glucose or YEPDA-sucrose medium in the form of stripes of about 3 cm² and were incubated at 17-20°C for 4 days in the presence of light. Then the carotenoids were evaluated in the biomass corresponding to 0.8 cm² from each stripe as indicated in example 2, selecting the stripes that exhibited an absorbance value greater than that of the VKPM Y-2476 parent strain. In this way the strain AST-A11 was selected, which had a higher astaxanthin yield than VKPM Y-2476 using glucose or sucrose as the source of carbon.

### EXAMPLE 6

### Genetic analysis of a series of strains of X. dendrorhous exhibiting different astaxanthin yield 6.1. Characterization of the extrachromosomal elements of X. dendrorhous

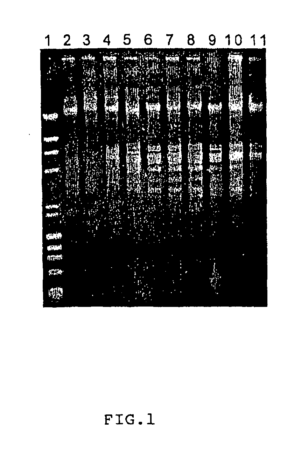

For the purpose of comparing a number of wild-type strains of *X*. *dendrorhous* with the astaxanthin-overproducing strains, their nucleic acids were analyzed in 0.8% agarose gel. The 4 wild-type strains analyzed were: CECT 1690 (also designated ATCC 24202 or CBS 5905), CECT 11028 (also designated ATCC 24203 or CBS 5908), CBS 6938 and ATCC 24229. For their part, the overproducing strains analyzed were as follows: VKPM Y-989, VKPM Y-2240, VKPM Y-2259, VKPM Y-2279, VKPM Y-2410 and VKPM Y-2476. Once the whole DNA of the aforementioned strains had been purified it was treated with RNase and was analyzed by electrophoresis. The result is shown in Fig. 1. As can be seen, the overproducing strains have a pattern of extrachromosomal nucleic acids that is different from that of the wild-type strains. The strain *X*. *dendrorhous* ATCC 24229 has the pattern that is the most similar to that of the overproducing strains.

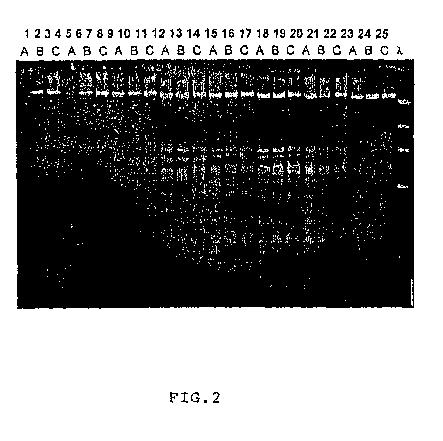

The presence of extrachromosomal genetic elements has been described in various strains of *X*. *dendrorhous*; these are mainly double-stranded RNAs (dsRNA's), which in some strains have been found to be encapsidated in virus-like particles (VLP) (Castillo A. and Cifuentes V. 1994. Curr. Genet. 26: 364-368; Pfeiffer, H. et al. (1996). Curr. Genet. 30: 294-297). With the aim of determining whether the extrachromosomal elements of the overproducing strains were molecules of dsRNA, they were treated with RNase A (350 ng/ml) at two different saline concentrations: SSC 0.01x and SSC 2x. In addition, a control sample was prepared without treating with RNase A in SSC 0.01x. The said samples were incubated for 30 minutes at 37°C and were then analyzed by electrophoresis in 0.8% agarose gel. As can be seen from Fig. 2, no extrachromosomal element was degraded after incubation with RNase A, therefore it was concluded that the said nucleic acids were not molecules of dsRNA.

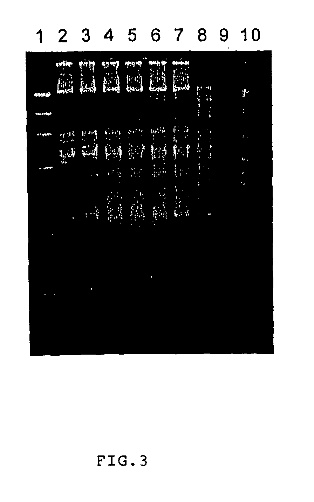

With the aim of determining the nature of the said extrachromosomal elements, a sample of whole DNA from *X*. *dendrorhous* Y-2410 was treated with various nucleic-acid-modifying enzymes: RNase A, RNase H, nuclease S1, DNase I and the restriction endonuclease *Bam*HI. The results are shown in Fig. 3. Digestion with RNase A confirmed the results described previously. However, (i) the complete disappearance both of the extrachromosomal elements and of the whole DNA in lane 9 (treatment with DNase I) and (ii) their susceptibility to digestion with the restriction endonuclease *Bam*HI (lane 10) showed that they were DNA molecules.

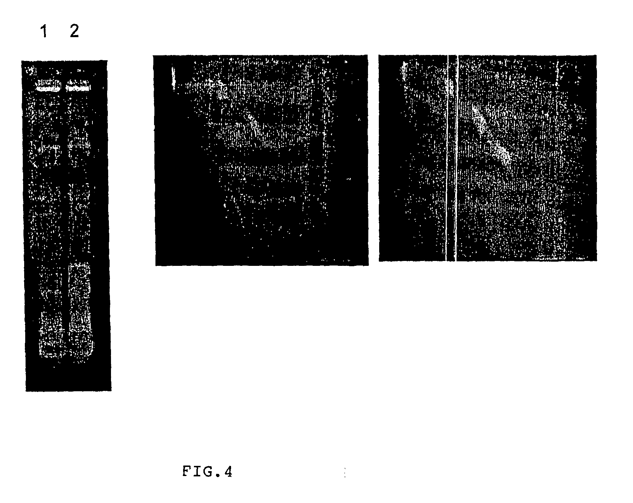

Next, determination of the conformation of the extrachromosomal elements in the strains *X*. *dendrorhous* CBS 6938 and *X*. *dendrorhous* Y-2410 was undertaken. For this, a sample of whole DNA was analyzed by two-dimensional electrophoresis on 1% agarose gel. As can be seen in Fig. 4, the pattern of extrachromosomal elements of both strains is the same in the two dimensions of the gel, i.e. the DNA fragments migrate according to their size. This indicates that the DNA molecules detected in *X*. *dendrorhous* possess linear conformation. It is concluded from these results that the extrachromosomal elements of the strains analyzed are linear plasmids formed from double-stranded DNA.

### 6.2. Determination of the genetic polymorphism of a series of strains of X. dendrorhous using the RAPD technique

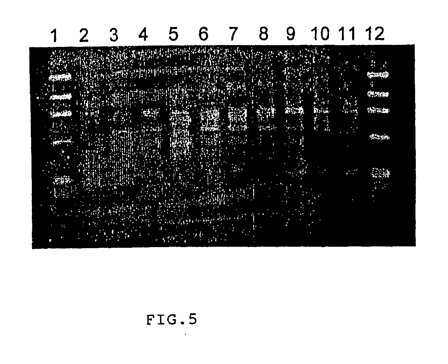

The objective was to determine the existence of genetic diversity in the different strains of *X*. *dendrorhous* by randomly amplifying their DNA by PCR. This technique is called RAPD (randomly amplified polymorphic DNA). The polymorphisms are detected owing to the presence of (i) changes (for example point mutations) in the primer binding sequence, (ii) changes in the DNA sequence (for example insertions and inversions) that give rise to changes in size, (iii) insertions that change the size of an amplified DNA fragment, (iv) disappearance of the binding site of the primer, etc. This study included the 4 wild-type strains and the 6 overproducing strains described in Section 6.1. The primer 5'-CATGTGTGGCGGGCA-3' previously described (Williams J.G. et al. 1990. Nucleic Acids Res. 18: 6531-6535), which had been used previously in *X*. *dendrorhous* (Meyer P.S. et al. 1994. Biotechnol. Tech. 8: 1-6), was used for the analysis. The whole DNA was denatured by heating for 5 minutes at 96°C and the PCR reaction was carried out in a final volume of 50 µl in a GeneAmp PCR System 2400 (Perkin Elmer). Thirty-five cycles of amplification were carried out in the following conditions: ring formation at 30°C for 1 minute, polymerization for 2 minutes at 72°C and denaturation for 1 minute at 92°C. In the last cycle the polymerization time was extended to 10 minutes at 72°C, followed by cooling to 4°C. The DNA fragments amplified by this technique were submitted to 1% agarose electrophoresis and the results obtained are shown in Fig. 5. As can be seen, the astaxanthin-overproducing strains generated an equivalent pattern, whereas the *X*. *dendrorhous* strain ATCC 24229 (which proved to be the most similar to the overproducing strains in the analysis performed in Section 6.1) had an amplification pattern different from that of the overproducing strains. It can be concluded from the experiments that the astaxanthin-overproducing strains differ genetically from all the wild-type strains analyzed.

### EXAMPLE 7

### Method of production of astaxanthin by flask fermentation of X. dendrorhous

Firstly, slants with R3-02-5 medium were seeded and were incubated for 5 days at 17-20°C. The composition of the R3-02-5 medium is as follows: bactopeptone 6.5 g/l, yeast extract 2 g/l, KH₂PO₄ 0.4 g/l, K₂HPO₄ 1.3 g/l, MgSO₄·7H₂O 1.3 g/l, (NH₄)₂SO₄ 1.3 g/l, NaCl 0.1 g/l, CaCl₂ 0.1 g/l, glucose 50 g/l, agar 20 g/l, H₃BO₃ 500 µg/l, CuSO₄ 40 µg/l, KI 100 µg/l, FeCl₃ 200 µg/l, MnSO₄·H₂O 400 µg/l, Na₂MoO₄·2H₂O 200 µg/l, ZnSO₄·7H₂O 400 µg/l, pH 6.0. Once the microorganism had grown, each slant was resuspended in 3 ml of saline solution and this suspension was used for seeding 500-ml flasks containing 25 ml of R4-062-7 medium at the rate of 1.5 ml of suspension of cells per flask. These inocula were incubated for 48 hours at 17-20°C and 250 rpm.

The inocula were used for seeding 500-ml flasks (3 indentations) containing 25 ml of R4-20 medium at the rate of 2.5 ml per flask. The composition of the R4-20 medium is as follows: yeast extract 6.2 g/l, cottonseed flour 5.5 g/l, KH₂PO₄ 2 g/l, K₂HPO₄ 0.4 g/l, MgSO₄·7H₂O 1.5 g/l, (NH₄)₂SO₄ 2 g/l, NaCl 0.2 g/l, CaCl₂ 0.2 g/l, CaCO₃ 1.6 g/l, glucose 200 g/l, biotin 50 µg/l, thiamine 500 µg/l, calcium pantothenate 2 mg/l, pH 5.8-6.0. These flasks were incubated at 17-20°C in the presence of light for 5-7 days. The yields of astaxanthin from the VKPM Y-2476 strain in these conditions were about 150 mg/l and 4000 ppm with a biomass of 37 g/l.

### EXAMPLE 8

### Improvement of the production of astaxanthin by modification of the culture medium

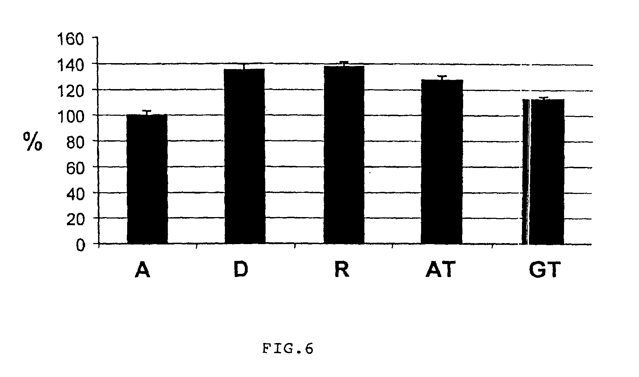

With the aim of improving the production of astaxanthin, addition of various compounds to the culture medium was tested: (i) agents that release free radicals such as duroquinone (25-50 µM), (ii) compounds that are inducers of carotenogenesis in other carotenoid-producing microorganisms, such as retinal (35 µM) and trisporic acids (50-100 µg/ml), (iii) molecules that are precursors of the hydrocarbon chain such as glutamate (5.5 mg/ml) instead of cottonseed flour. Fermentation was carried out as described in example 7 and the results obtained are shown in Fig. 6. The astaxanthin yields obtained in the best conditions were 225 mg/l and 5000 ppm with a biomass of 45 g/l. As can be seen, all these compounds gave rise to increases in yield relative to the original culture medium R4-20.

### EXAMPLE 9

### Development of a culture medium that increases the specific yield of astaxanthin

With the aim of increasing the specific yield of astaxanthin (mg of astaxanthin / g of biomass), a culture medium was developed using the GALOP software based on genetic algorithms, developed by Weuster-Botz D. (Institut für Biotechnologie, Forschungszentrum Jülich GmbH, Jülich, Germany). The design was based on keeping the concentrations of some of the components of the culture medium constant (KH₂PO₄, K₂HPO₄, MgSO₄·7H₂O, NaCl, CaCl₂ and CaCO₃) and analysing variable concentrations of other components (glucose, glycerol, peptone, corn steep solid (CSS), ammonium sulphate and soya oil). Using the GALOP software, 7 culture media were designed, which consisted of the same components but in variable concentrations. Table I shows the composition of the culture media analyzed in the 4th generation.

**Table I**

| Culture medium | Glucose 50% (ml) | Glycerol 50% (µl) | Peptone 10% (ml) | CSS 10% (ml) | (NH₄)₂SO₄ 10% (µl) | Soya oil (ml) | Salts 25x (ml) | Water (ml) |
|---|---|---|---|---|---|---|---|---|
| P | 10.0 | 5 | 1.5 | 1.4 | 500 | 0.1 | 3 | 8.50 |
| 1 | 9.9 | 2621 | 1.6 | 1.4 | 572 | 0.1 | 3 | 8.50 |
| 2 | 6.7 | 1987 | 1.5 | 1.4 | 1746 | 1.6 | 3 | 7.06 |
| 3 | 10.0 | 5 | 1.5 | 0.2 | 762 | 0.1 | 3 | 9.43 |
| 4 | 6.9 | 325 | 1.9 | 1.7 | 117 | 0.1 | 3 | 10.96 |
| 5 | 10.0 | 639 | 1.5 | 0.4 | 1778 | 0.1 | 3 | 7.58 |
| 6 | 9.9 | 4683 | 1.9 | 0.2 | 508 | 0.1 | 3 | 4.76 |

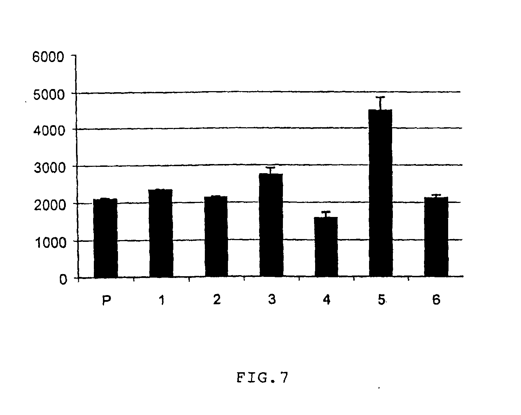

The salt solution 25x contained per litre: 50 g of KH₂PO₄, 10 g of K₂HPO₄, 37.5 g of MgSO₄·7H₂O, 5 g of NaCl, 5 g of CaCl₂ and 40 g of CaCO₃. Fig. 7 shows the results for specific yield obtained with these culture media after carrying out 4 successive generations of optimization of their composition. As can be seen, we succeeded in developing a culture medium that increased the specific yield of astaxanthin of the strain *X*. *dendrorhous* Y-2410 from 2096 ppm (µg/g) (culture medium P) to 4490 ppm (culture medium 5) without a drop in absolute yield (mg/l).

### EXAMPLE 10

### Improvement of the production of astaxanthin by illuminating the culture

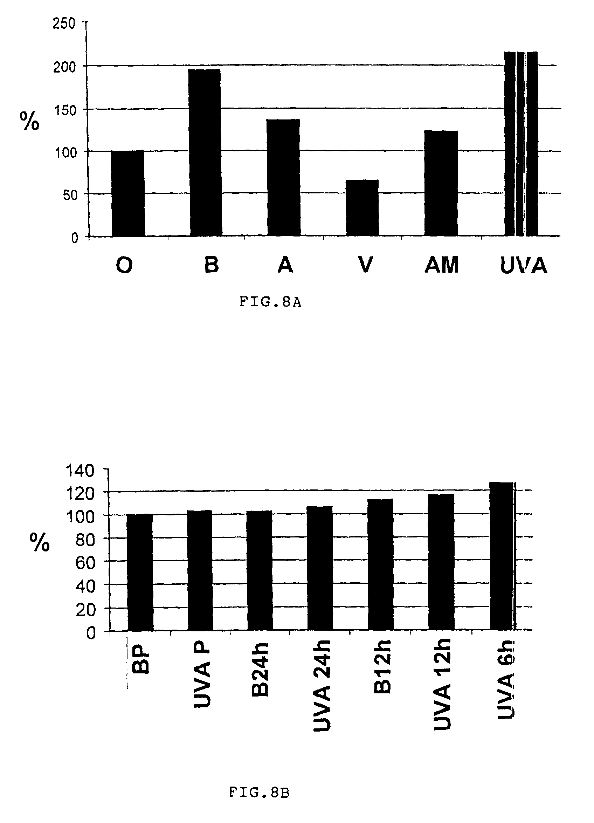

With the aim of developing improved methods of production of astaxanthin, the Y-2476 strain was fermented in a flask while varying the wavelength of the illumination used. For this, a series of fermentations was carried out, as described in example 7. The flasks were incubated at 17-20°C in the presence or in the absence of light for 6 days. In addition, different types of light were tested: white, blue, green, yellow and ultraviolet. The results obtained are shown in Fig. 8A. As can be seen, both white light and ultraviolet light gave rise to the most significant increases in production relative to the fermentations effected in darkness.

Furthermore, the production of astaxanthin from the Y-2476 strain was compared in flask fermentations illuminated permanently or with cycles of illumination / darkness of 6, 12 or 24 hours. These cycles were effected both with white light and with ultraviolet light. The results obtained are shown in Fig. 8B. As can be seen, the cycles of 6 hours of ultraviolet light / darkness gave rise to the most significant increases in production.

### EXAMPLE 11

### Method of production of astaxanthin by fermentation of X. dendrorhous in a 10-litre fermenter

The flasks of inoculum (2 litres with 200 ml of R4-062-7 medium, example 1) were seeded with the cells from one slant per flask and were incubated at 20°C under illumination with orbital agitation at 250 rpm for 48 hours. In this way a culture medium was obtained with 7% of biomass (expressed as pellet cell volume, PCV) and a pH of about 3. Next, an intermediate fermenter for vegetative growth was inoculated with R4-10-3P medium with 0.4% (v/v) of the inoculum. The R4-10-3P medium has the following composition per litre: yeast extract 6.2 g, cottonseed flour 5.5 g, glucose 100 g, KH₂PO₄ 2 g, K₂HPO₄ 0.4 g, MgSO₄·7H₂O 1.5 g, PO₄H(NH₄)₂ 5 g, NaCl 0.2 g, CaCl₂·2H₂O 0.4 g and thiamine 0.5 mg, at a final pH of 5.8-6.0. The vegetative stage was incubated at 20°C for 48 hours with sufficient agitation to maintain at least 50% dissolved oxygen (DO₂), aeration at 1.5 vvm (volume/volume/minute) and control of pH to 4.5 with ammonia at a concentration of 12.5%. The growth obtained in the vegetative phase reached 30-35% of biomass (expressed as PCV). The production phase was carried out in a fermenter with a borosilicate glass tank with R4-10 medium, which has the following composition relative to the volume after seeding (9 litres): yeast extract 6.2 g/l, cottonseed flour 5.5 g/l, KH₂PO₄ 2 g/l, K₂HPO₄ 0.4 g/l, MgSO₄·7H₂O 1. 5 g/l, (NH₄)₂HPO₄ 5 g/l, NaCl 0.2 g/l, CaCl₂·2H₂O 0.2 g/l, solution of trace elements 2 ml/l and polypropyleneglycol 2025 0.9 g/l, adjusting the pH to 5.4 with NaOH. The composition of the solution of trace elements is as follows: boric acid 1 mg, sodium molybdate dihydrate 0.4 mg, zinc sulphate heptahydrate 0.8 mg, ferric chloride hexahydrate 0.4 mg, copper sulphate pentahydrate 0.8 mg, potassium iodide 0.2 mg, manganese sulphate tetrahydrate 0.8 mg. The medium was adjusted to a volume of 6 litres and, after sterilization, 1.05 litres of glucose syrup (to give a glucose concentration of 100 g/l after seeding) and 1 ml/l of solution of vitamins (biotin 0.1 g/l, thiamine 1 g/l and calcium pantothenate 4 g/l) were added. The fermenter was inoculated with 2 litres of the vegetative culture and was adjusted to the following vegetative culture conditions: (I) Aeration: 1.5 vvm. (II) Stirring: 700 rpm up to 18 hours, then increasing to 1200 rpm to maintain DO₂ > 50%. (III) Pressure: 0 kg/cm². (IV) Temperature: 20°C up to 72 hours and then 17°C. (V) pH: control of lower limit to 4.5 with 12.5% ammonia up to 72 hours, then lowering the limit to 3.0. (VI) DO₂: control with lower limit at 50% by stirring. (VII) Illumination: 6 fluorescent tubes arranged above the surface of the tank with a total power of 80 watts.

The following additions were made: (I) Glucose in 50% solution in accordance with the following schedule of addition (expressed as grams of pure glucose per litre per hour): 0 between hours 0-25, 2.5 between hours 26-51, 2.3 between hours 52-116 and 1.6 between hours 117-212. The total consumption of 50% glucose in additions was 6.47 litres. (II) Absolute ethanol: 0.5% every 24 hours starting from 72 hours. The total consumption was 270 ml. (III) Antifoaming agent: polypropyleneglycol (PPG), with a total consumption of 200 ml. (IV) 12.5% ammonia for control of pH, with a total consumption of 464 ml.

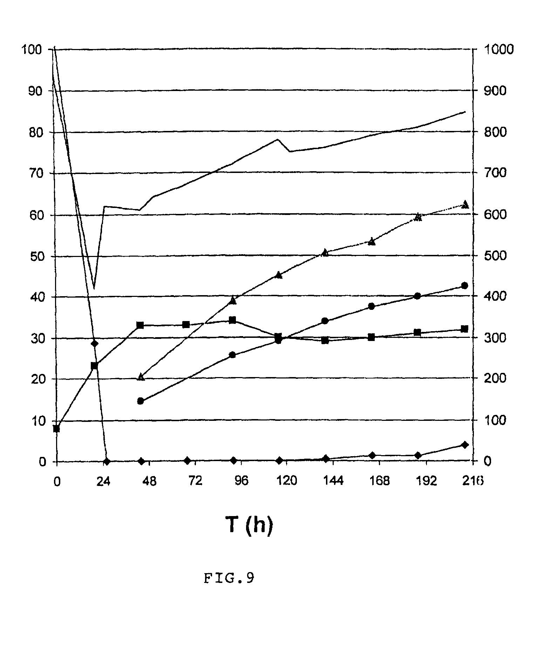

Moreover, the necessary partial harvests were effected to maintain the working volume at 10 litres. The total volume of partial harvests was 2.6 litres and the final volume of the tank was 8.5 litres. Fermentation ended at 212 hours with a biomass of 32% (expressed as PCV) which was equivalent to 84 g/l (expressed as dry weight). The concentration of astaxanthin determined by HPLC was 425 mg/l, so that the astaxanthin strength of the dry biomass exceeded 5000 ppm. The total concentration of accumulated carotenoids was 623 mg/l (7416 ppm). The evolution of fermentation is shown in Fig. 9.

### EXAMPLE 12

### Method of production of astaxanthin by the fermentation of X. dendrorhous in an 800-litre fermenter

The inoculum culture medium (R4-062-7, example 1) was prepared in 2000-ml Erlenmeyer flasks at a rate of 200-400 ml per flask. Once sterilized they were seeded with *X*. *dendrorhous* and then incubated at 20°C and 250 rpm for 48 hours. The inoculum was transferred in sterile conditions in a proportion of 0.4% (v/v) to an intermediate tank for vegetative growth with R4-10-2 culture medium, which has the following composition per litre: yeast extract 6.2 g, Pharmamedia 5.5 g, glucose syrup (70% w/w) 143 g (sterilized separately), corn steep solid 24 g, monopotassium phosphate 2 g, dipotassium phosphate 0.4 g, magnesium sulphate heptahydrate 1.5 g, diammonium phosphate 5 g, sodium chloride 0.2 g, calcium chloride dihydrate 0.4 g, antifoaming agent 0.1 g, thiamine hydrochloride 1 mg, boric acid 1 mg, sodium molybdate dihydrate 0.4 mg, zinc sulphate heptahydrate 0.8 mg, ferric chloride hexahydrate 0.4 mg, copper sulphate pentahydrate 0.8 mg, potassium iodide 0.2 mg, manganese sulphate tetrahydrate 0.8 mg. Its initial pH was 5.6. The vegetative phase was incubated at 17°C for 54 h, with aeration of 1.5 v/v/m and a head pressure of 1 atmosphere, until a pH below 3.5 was reached. Then a second growth phase was effected in the R4-10-2 culture medium until a biomass of 30-35% (expressed as PCV) was achieved. The production fermenter (800 litres capacity) containing R4-10-2 medium was seeded with 20% (v/v) of the vegetative culture.

Fermentation was carried out with the following temperature programme: 19°C to 16 hours, 18°C from 16 to 60 hours and 17°C until the end. Stirring varied between 150 and 275 rpm, with aeration of 1.5 v/v/m. The head pressure was maintained at 0.5 atm. Control of pH was effected with 25% ammonium hydroxide, it being kept above 4.5 until 24 hours, above 3.5 from 24 to 60 hours and above 3.0 from 60 hours until the end. Dissolved oxygen was maintained in a range above 50%, increasing the stirring rate when necessary. During fermentation, additions of glucose were made (expressed in kg/m³/hour) in accordance with the following programme: 0 between hours 0-24, 4.41 between hours 24-48, 3.77 between hours 48-70, 2.08 between hours 70-94 and 2.60 between hours 94-184.

Furthermore, the following were carried out when necessary: (i) additions of antifoaming agent and (ii) partial harvests for maintaining the volume in the fermenter at around 75% of its total capacity. The culture was illuminated by means of a 1-metre long borosilicate tube submerged in the culture that contained six 54-watt fluorescent tubes (total 324 W). Fermentation continued for 184 hours, at the end of which a yield of astaxanthin of 400 mg/l was obtained, and biomass of 90 g/l (expressed as dry weight), which means an astaxanthin strength in the dry biomass above 4400 ppm.

Addition of 50 µM of duroquinone resulted in increases in astaxanthin yield of around 15-20%.

### EXAMPLE 13

### Process for recovery of the astaxanthin-rich biomass of X. dendrorhous and analysis of bioavailability in trout

The yeast *X*. *dendrorhous,* fermented in pure culture as described in the previous examples, was transferred from the fermenter to a refrigerated tank where it remained until it was recovered by centrifugation or filtration. Preliminary concentration of the culture medium was effected continuously by means of centrifuges or filtration units, giving rise to concentrated culture media. For the purpose of protecting the astaxanthin during processing of the biomass, in some cases the antioxidant ethoxyquin was added to the concentrated biomass. Next, drying of the concentrated biomass was carried out, using conventional methods, in such a way that the majority of the cells were not disrupted during the process. Finally the dry biomass was stored in such a way that it was protected from light, oxygen and moisture.

The dry biomass was mixed with a feed preparation used in fish farming, in such a way that the astaxanthin concentration in the mixture was 75 ppm. Then the mixture was extruded in the form of cylinders or pellets, with which a number of trout were fed for two months. Then 10 trout selected at random were sacrificed and the content of astaxanthin and carotenoids in their muscle tissue was determined. In addition, the content of astaxanthin and carotenoids was analyzed in another 10 control trout that had not been fed with the pellets previously described. The average values obtained are shown in the following table:

| | Trout fed with dry biomass | Control trout not fed with dry biomass |
|---|---|---|
| Astaxanthin (µg/g) | 3.5 | 0.0 |
| Carotenoids (µg/g) | 6.8 | 0.1 |

The results of the bioavailability test show that the astaxanthin and the other carotenoids present in the dry biomass of *X*. *dendrorhous* were transferred to the tissues of the trout, endowing them with an attractive salmon coloration.

### Detailed description of the diagrams

**Fig. 1.** Photograph of an agarose gel, showing the whole DNA of a number of strains of *X*. *dendrorhous* exhibiting different yields of astaxanthin. *Lane 1*: Molecular weight marker of DNA, the bands of which have the following sizes expressed in base pairs: 23 130, 9416, 6557, 4361, 2322, 2027, 1353, 1078, 872, 603 and 300. *Lane 2*: *X*. *dendrorhous* CECT 1690 (also designated ATCC24202 or CBS 5905). *Lane 3*: *X*. *dendrorhous* CECT 11028 (also designated ATCC 24203 or CBS 5908). *Lane 4: X*. *dendrorhous* CBS 6938. *Lane 5*: *X*. *dendrorhous* ATCC 24229. *Lane 6*: *X*. *dendrorhous* Y-989. *Lane 7*: *X*. *dendrorhous* Y-2240. *Lane 8*: *X*. *dendrorhous* Y-2259. *Lane 9*: *X*. *dendrorhous* Y-2279. *Lane 10*: *X*. *dendrorhous Y*-2410. *Lane 11*: *X*. *dendrorhous* Y-2476. The whole DNA (size larger than 23 kb) is shown at the top of the diagram, with the extrachromosomal elements given below it (size between 2.3 and 9 kb).
**Fig. 2.** Photograph of an agarose gel, showing the whole DNA of a number of strains of *X*. *dendrorhous* in three different conditions: (A) Control (SSC 0.01x, without treatment with RNase A). (B) Treated with RNAse A at a concentration of 350 ng/ml in SSC 0.01x. (C) Treated with RNase A at a concentration of 350 ng/ml in SSC 2x. *Lanes 1-3*: *X*. *dendrorhous* CECT 1690 (also designated ATCC 24202 or CBS 5905). *Lanes 4-6*: *X*. *dendrorhous* CECT 11028 (also designated ATCC 24203 or CBS 5908). *Lanes 7-9*: *X*. *dendrorhous* CBS 6938. *Lanes 10-12*: *X*. *dendrorhous* ATCC 24229. *Lanes 13-15*: *X*. *dendrorhous* Y-989. *Lanes 16-18*: *X*. *dendrorhous* Y-2240. *Lanes 19-21*: *X*. *dendrorhous* Y-2410. *Lanes 22-24*: *X*. *dendrorhous* Y-2476. *Lane 25*: Molecular weight marker of DNA, the bands of which have the following sizes expressed in base pairs: 23 130, 9416, 6557, 4361, 2322 and 2027.
**Fig. 3.** Photograph of an agarose gel, showing the whole DNA of *X*. *dendrorhous* Y-2410 treated with various nucleic-acid-modifying enzymes. *Lane 1*: Molecular weight marker of DNA, the bands of which have the following sizes expressed in base pairs: 23 130, 9416, 6557, 4361, 2322, 2027, 1353 and 1078. *Lane 2*: Control DNA, untreated. *Lane 3*: Treatment with RNase A in 2x SSC. *Lane 4*: Treatment with RNase A in 1x SSC. *Lane 5:* Treatment with RNase A in 0.02x SSC. *Lane 6*: Treatment with RNase A in 0.01x SSC. *Lane 7*: Treatment with RNase H. *Lane 8*: Treatment with nuclease S1. *Lane 9*: Treatment with DNase I. *Lane 10*: Digestion with the restriction endonuclease *Bam*HI.
**Fig. 4.** *Left*: agarose gel of the strains *X*. *dendrorhous* CBS 6938 (lane 1) and *X*. *dendrorhous* Y-2410 (lane 2). *Centre:* second dimension of the electrophoresis of lane 1 corresponding to *X*. *dendrorhous* CBS 6938. *Right*: second dimension of the electrophoresis of lane 2 corresponding to *X*. *dendrorhous* Y-2410.
**Fig. 5.** Photograph of an agarose gel, showing the DNA of a number of strains of *X*. *dendrorhous* amplified using the RAPD (randomly amplified polymorphic DNA) technique. *Lanes 1* and *12*: Molecular weight marker of DNA, the bands of which have the following sizes expressed in base pairs: 1353, 1078, 872, 603 and 300. *Lane 2*: *X*. *dendrorhous* CECT 1690 (also designated ATCC 24202 or CBS 5905). *Lane 3*: *X*. *dendrorhous* CECT 11028 (also designated ATCC 24203 or CBS 5908). *Lane 4*: *X*. *dendrorhous* CBS 6938. *Lane 5*: *X*. *dendrorhous* ATCC 24229. *Lane 6*: *X*. *dendrorhous* Y-989. *Lane 7*: *X*. *dendrorhous* Y-2240. *Lane 8*: *X*. *dendrorhous* Y-2259. *Lane 9*: *X*. *dendrorhous* Y-2279. *Lane 10*: *X*. *dendrorhous* Y-2410. *Lane 11*: *X*. *dendrorhous* Y-2476.
**Fig. 6.** Production of astaxanthin (ordinate) by flask fermentation of the strain *X*. *dendrorhous* Y-2476, adding the following compounds to the fermentation medium: duroquinone -D-, retinal -R-, trisporic acids -AT- or glutamate -GT-. The yield is expressed as a percentage relative to the standard condition -P- (100%).
**Fig. 7.** Specific yield of astaxanthin from *X*. *dendrorhous* Y-2410 in 7 different culture media designed using the GALOP program, which is based on genetic algorithms. The culture media designated P, 1, 2, 3, 4, 5 and 6 are shown on the abscissa. The ordinate shows the values of specific yield expressed in ppm (µg/g).
**Fig. 8.** Production of astaxanthin by flask fermentation of the strain *X*. *dendrorhous* Y-2476. **(A)** Illuminating the culture with different types of light: Darkness -O-, white -B-, blue -A-, green -V-, yellow -AM- and ultraviolet -UV-; **(B)** Illuminating the culture with different cycles of white or ultraviolet light: Continuous white -BP-; continuous UVA -UVAP-; White 24 hours -B24h-; UVA 24h-; White 12 hours -B12h-; UVA 12h; UVA 6h. The yield is expressed as a percentage relative to the standard condition (100%).
**Fig. 9.** Evolution of the yield of astaxanthin during fermentation of the strain *X*. *dendrorhous* Y-2476 in a 10-litre fermenter. Ordinate (left): PCV (%) ■, DO2 (%) - and glucose (g/l) ◆. Ordinate (right): astaxanthin HPLC (mg/l) •, carotenoids (mg/l) ▲, Abscissa: time, hours.

## Claims

1. A method of production of astaxanthin by fermentation, **characterized by** the cultivation of the strains of *X*. *dendrorhous* that are shown in Scheme 2 of the description or their mutants or transformed derivatives capable of producing, in a flask, at least 4000 ppm of astaxanthin at days 6-7 of fermentation.

2. A method of fermentation according to claim 1, **characterized in that**, in industrial fermentation, a yield of at least 5000 ppm of astaxanthin is reached at days 7-9 of fermentation.

3. A method of fermentation according to claims 1 and 2, **characterized in that** duroquinone is added during the fermentation process.

4. A method of fermentation according to claim 3, **characterized in that** duroquinone is added at a concentration of 25-50 µM.

5. A method of fermentation according to claims 1 and 2, **characterized in that** retinal is added during the fermentation process.

6. A method of fermentation according to claim 5, **characterized in that** retinal is added at a concentration of 35 µM.

7. A method of fermentation according to claims 1 and 2, **characterized in that** trisporic acids are added during the fermentation process.

8. A method of fermentation according to claim 7, **characterized in that** the trisporic acids are added at a concentration of 50-100 µg/ml.

9. A method of fermentation according to claims 1 and 2, **characterized in that** glutamate is added during the fermentation process.

10. A method of fermentation according to claim 9, **characterized in that** glutamate is added at a concentration of 5.5 mg/ml.

11. A method of fermentation according to claims 1 and 2, **characterized in that** medium 5 described in Table I of the description is used for the fermentation process.

12. A method of fermentation according to claim 11, **characterized in that** 4490 ppm of astaxanthin is produced in a flask at days 6-7 of fermentation.

13. A method of fermentation according to claims 1 to 12, **characterized in that** the fermentation medium is illuminated during the fermentation process.

14. A method of fermentation according to claim 13, **characterized in that** the source of illumination used is white light.

15. A method of fermentation according to claim 13, **characterized in that** the source of illumination used is ultraviolet light.

16. A method of fermentation according to claims 13 to 15, **characterized in that** illumination is carried out from the start to the end of fermentation, preferably between hours 40 and 200.

17. A method of fermentation according to claim 16, **characterized in that** cycles of 6 hours of illumination / darkness are used.

18. A method of fermentation according to any of the claims 1 to 17, **characterized in that**:
(a) Inocula of *X*. *dendrorhous* are seeded.
(b) The inocula of *X*. *dendrorhous* are cultivated for 48 hours at 20°C.
(c) Phases of primary culture of *X*. *dendrorhous* are seeded with about 0.4% (v/v) of the inoculum phase.
(d) The primary phases of *X*. *dendrorhous* are cultivated for 48-54 hours at 17-20°C.
(e) Each fermenter is seeded with 20% (v/v) of the primary phases of *X*. *dendrorhous*.
(f) The fermentation is incubated for 60-72 hours at 18-20°C and for the other 5-7 days at 17°C.

19. A method of fermentation according to claim 18, **characterized in that** at least 425 mg/l of astaxanthin is produced on days 7-9.

20. A method of fermentation according to claims 18 and 19, **characterized in that** concentrations of biomass are produced of at least 50 grams of dry weight of cells per litre of fermentation medium.

21. A method of fermentation according to claim 20, **characterized in that** concentrations of biomass are produced of at least 80 grams of dry weight of cells per litre of fermentation medium.

22. A method of fermentation according to claims 18 to 21, **characterized in that** at least 5000 µg of astaxanthin is produced per gram of dry weight of cells on days 7-9 of fermentation.

23. Biomass of *X*. *dendrorhous* with nutrient and pigmenting value, obtainable by the fermentation process described in claims 1 to 22, for use in food for humans and animals.

24. Biomass according to claim 23, **characterized in that** it contains:
a) A concentration of at least 5000 µg/g of astaxanthin;
b) A concentration of at least 7400 µg/g of total carotenoids;
c) A concentration of at least 15% of proteins and
d) A concentration of at least 15% of carbohydrates.

25. Compounds for animal food that consist of or contain the biomass of claims 23 and 24.

26. Compounds for human food that consist of or contain the biomass of claims 23 and 24.
